# EUROPÄISCHE PATENTSCHRIFT

(11) **EP 0 419 830 B1**
(45) Veröffentlichungstag und Bekanntmachung des Hinweises auf die Patenterteilung: **23.02.1994**
(21) Anmeldenummer: 90115632.3
(22) Anmeldetag: 16.08.1990
(51) Int. Cl.: G01N 33/36, D01G 15/46

(54) **Auftrennvorrichtung**
Separating device
Dispositif de séparation

(30) Priorität: 29.08.1989 CH 3135/89
(43) Veröffentlichungstag der Anmeldung: 03.04.1991
(73) Patentinhaber: ZELLWEGER LUWA AG, CH-8610 Uster (CH)
(72) Erfinder: Beeler, Alfred, CH-8810 Horgen (CH)

(56) Entgegenhaltungen:
- DE-C- 909 549
- FR-A- 1 341 775
- FR-A- 2 388 914
- US-A- 3 370 326
- DE TEX, Band 27, Juni 1968, Seiten 353-354

## Beschreibung

Die Erfindung bezieht sich auf eine Auftrennvorrichtung gemäss dem Oberbegriff des Anspruchs 1.

Für die qualitative und quantitative Prüfung von textilen Fasern existieren zahlreiche Verfahren und Apparaturen. Bei vielen Prüfverfahren kann das ungeordnete Fasermaterial, z.B. Baumwolle in Flockenform, nicht sofort gemessen werden, sondern ist zunächst in Faserbänder mit parallelisierter Faserlage aufzubereiten. Solche vorparallelisierte Faserbänder dienen somit als Vorlage für alle Prüfgeräte die als Prüfgut Fasern mit parallelisierter Faserlage benötigen.
Das sind zum Beispiel:
- Faserrichter zur Endenordnung von Faserbärten.
- Bürststationen für Faserbärte ohne Endenordnung.
- Zugprüfgeräte zur Bestimmung der Bündelreissfestigkeit und Dehnung.
- Optische Scanner, wie Bilddatenanalysegeräte.
- Faserreifeprüfgeräte.
- Faserfeinheitsprüfgeräte.
- Faserklassiergeräte etc.

Bei solchen Geräten besteht das Bedürfnis das herzustellende Faserband mehrfach von einer Bearbeitungstrommel wieder abzulösen um es einem weiteren Bearbeitungszyklus zu unterwerfen. Bei bekannten, manuelle betriebenen Geräten dieser Art [siehe z.B. Sonderdruck G.Spiridonow in Melliand Textilberichte 59 (1978)] versteht sich von selbst, dass das Auftrennen der herzustellenden Faserbänder sehr zeitraubend und wenig reproduzierbar ist. Der Handbetrieb erfordert ein hohes Geschick und bewirkt zudem eine ständige Berührung des Rohstoffes mit der blossen Haut, so dass eine Kontamination mit Schweiss nicht verhindert werden kann.

Hier will die Erfindung Abhilfe schaffen. Der Erfindung liegt die Aufgabe zugrunde, eine universell einsetzbare Auftrennvorrichtung für auf Bearbeitungstrommeln aufgewickelte Faservliese mit parallelisierter Faserlage aus Wirrfaserkollektiven zu schaffen, welche vollautomatisch und ohne Faserbeschädigung arbeitet.

Die Erfindung löst die gestellte Aufgabe mit einer Vorrichtung, welche die Merkmale des Anspruchs 1 aufweist.

Die durch die Erfindung erreichten Vorteile sind im wesentlichen darin zu sehen, dass dank der erfindungsgemässen Vorrichtung Faserbänder mit parallelisierter Faserlage vollautomatisch aufgetrennt und von den hierzu verwendeten Bearbeitungstrommeln abgewickelt werden können.

Die erfindungsgemässe Auftrennvorrichtung kann wahlweise mit einer einzigen Bearbeitungstrommel oder mehreren Bearbeitungstrommeln verknüpft werden. Ein Beispiel für den Einsatz mit zwei eine Garnitur aufweisenden Bearbeitungstrommeln ist in der unter der Nummer EP-A-0 393 360 veröffentlichten europäischen Patentanmeldung näher beschrieben. Die Bearbeitungstrommeln für das Faservlies können statt einer Garnitur auch eine Vakuumsaugvorrichtung oder eine elektrostatische Haltevorrichtung aufweisen um das zu bearbeitende Faservlies auf der Bearbeitungstrommel temporär festzuhalten.

Ein Ausführungsbeispiel der Erfindung ist in den Zeichnungen dargestellt und wird im folgenden näher beschrieben.
Fig. 1 stellt einen schematischen Querschnitt durch eine erfindungsgemässe Vorrichtung dar, welche mit einer einzigen Bearbeitungstrommel zusammenwirkt;
Fig. 2 stellt einen zur Fig. 1 senkrechten Längsschnitt durch den Niederhalter und die Garnitur der erfindungsgemässen Vorrichtung dar;
Fig. 3 stellt einen schematischen Querschnitt durch die erfindungsgemässe Vorrichtung dar, mit einer zusätzlichen Einlegevorrichtung für das aufgetrennte Faserband; und
Fig. 4 stellt einen schematischen Querschnitt durch die erfindungsgemässe Vorrichtung nach Fig. 3 mit ausgefahrener Einlegevorrichtung dar.

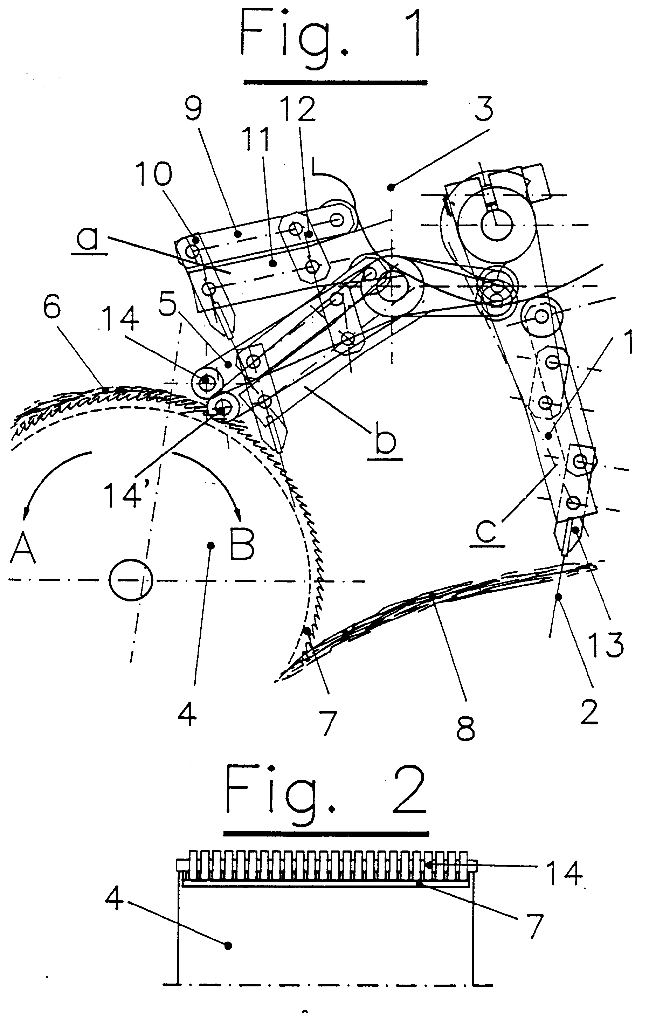

In Fig. 1 ist eine bevorzugte erfindungsgemässe Auftrennvorrichtung dargestellt, welche mit einer in beiden Drehrichtungen A und B wahlweise (mit nicht dargestellten Antriebsmitteln) antreibbare, zylindrische Bearbeitungstrommel 4 mit einer am Zylindermantel angebrachten Garnitur 7 zusammenwirkt. Die Garnitur 7 besteht aus einem Sägezahnprofil; sie kann aber auch aus einer Vielzahl von Häkchen oder Nädelchen bestehen.
Das sich in Wirrlage befindliche, vorportionierte Fasermaterial wird dabei mittels einer (nicht dargestellten) Zuführvorrichtung auf die Bearbeitungstrommel 4 gebracht.
Der von und zur Bearbeitungstrommel 4 hin oszillierbar angeordneten Arm 1 besteht aus vier gelenkartig miteinander verbundenen Balken 9,10,11,12, wobei der eine der vier Balken 10 mit einem Trennkamm 13 versehen ist, der eine Mehrzahl von Nadeln 2 aufweist. Das aus den vier Balken 9,10,11,12 bestehende Hebelwerk ist längs einer Trennkurve 3 beweglich angeordnet. Die Form dieser Trennkurve 3 ist empirisch zu bestimmen und hängt von allen übrigen Vorrichtungparametern ab (z.B. Dimension der Bearbeitungstrommel 4, Länge des Armes 1, Art des Faservlieses 6, Position und Dimension der, der Auftrennvorrichtung nachgelagerten Bearbeitungsstationen und die nachstehend beschriebene, variable Stellung der Nadeln 2 des Trennkamms 13). Sie ist derart zu wählen, dass die Auftrennung des auf der Bearbeitungstrommel 4 aufgewickelten Faservlieses 6 an einer definierten Stelle und die Abwicklung des aufgetrennten Faservlieses 6 von der Bearbeitungstrommel 4 mit dem einen freien Ende 8 des Faservlieses 6 längs einer Mantelerzeugenden der Bearbeitungstrommel 4 bewirkt wird.

Die Bewegung des Armes 1 und dessen Koordination mit der Position der vor- und nachgelagerten Bearbeitungsstationen erfolgt automatisch mittels einer (nicht dargestellten) Steuerungsvorrichtung.

Position a) in Fig. 1 zeigt den Arm in seiner Ruhestellung. Der Arm 1 wandert nun längs der Trennkurve 3 gegen die Bearbeitungstrommel 4 zu, wobei sich durch das Hebelwerk die Winkelstellung der Nadeln 2 des Trennkammes 13 derart verändert, dass die Nadeln 2 tangential zur Mantelfläche in die Garnitur 7 auftreffen, dabei das darauf liegende Faservlies 6 durchstechen und es von der Bearbeitungstrommel 4 abheben. Dabei wird das Faservlies 6 zwischen dem als Rolle ausgebildeten Niederhalter 5 und den Nadeln 2 unterbrochen (Stellung b). Die Trennkurve 3 und das Hebelwerk 9,10,11,12 des Trennkamms 13, das in der bevorzugten Ausführungsform ein Gelenkviereck ist, schwenkt nun den Trennkamm 2 in solcher Weise, dass das Kopfende des Faservlieses 8 aus den Nadeln 2 gleitet (Stellung c).
Während des Trennvorganges wird das Schleppende des Vlieses durch den aus der Ruheposition 14 in die Arbeitsstellung 14′ gewanderten Niederhalter 5 an die Bearbeitungstrommel 4 gepresst. Der Niederhalter 5 weist an seinem freien Ende eine Rolle auf, welche mit einem weichen Belag, z.B. aus Gummi versehen ist. In einer bevorzugten Ausführungsform (Fig. 2) ist die Rolle des Niederhalters 5 wellenartig ausgebildet, wobei die Vertiefungen der Welle mit den Garnituren 7 der Bearbeitungstrommel 4 korrespondieren.

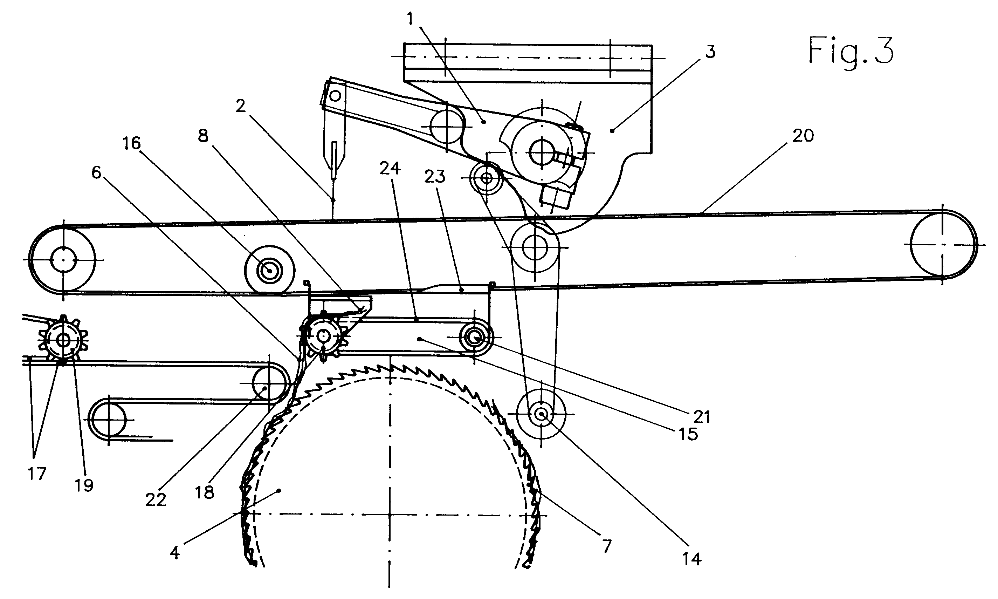

Das durch den Trennvorgang vom Umfang der Bearbeitungstrommel 4 abstehende Kopfende 8 des Faservlieses 6 kann nun einer beliebigen Bearbeitungsstation (z.B. Streckwerk) zugeführt werden. Zweckmässigerweise wird dabei das Kopfende 8 des aufgetrennten Faservlieses 6, wie in Fig. 3 dargestellt, in eine Einlegevorrichtung 15-24 eingelegt. Die Problematik dieses Vorganges besteht darin, dass die Fasern des Kopfendes 8, welche sich am Ende des Auftrennvorgangs in den Nadeln 2 befinden, beim Einlegen in die Einlegevorrichtung 15-24 nicht umgelegt werden dürfen, d.h. es dürfen dabei keine Häkchen entstehen.

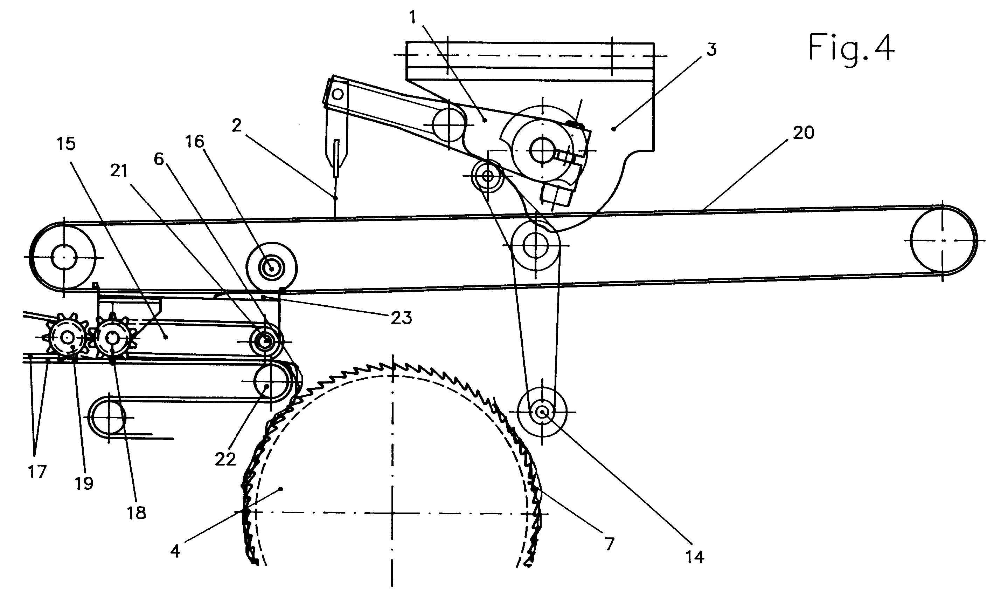

Zur Lösung dieses Problems eignet sich eine in den Fig. 3 und 4 im Detail dargestellte Einlegevorrichtung 15 - 24, welche im wesentlichen aus einem Einlegeschlitten 15 besteht, der an einem oberen Transportband 20 hängt. Der Einlegeschlitten 15 besteht aus einem mittleren Transportband 24, welches über das Zahnrad 18 angetrieben wird. In der Ruhestellung befindet sich der Einlegeschlitten 15 im rechten Anschlag des oberen Transportbandes 20. Nachdem das Faservlies 6 aufgetrennt in den Nadeln 2 hängt, wird der Einlegeschlitten 15 mittels des oberen Transportbandes 20 bis an das Faservlies 6 herangefahren. Anschliessend wird die Trommel 4 im Gegenuhrzeigersinn gedreht bis das Faservlies 6 aus den Nadeln 2 gleitet und auf das mittlere Transportband 24 des Einlegeschlittens 15 zu liegen kommt, wie in Fig. 3 dargestellt.
Danach wird der Einlegeschlitten 15 - wie in Fig. 4 dargestellt - in den linken Anschlag des oberen Transportbandes 20 gefahren, wo die Zahnräder 18 und 19 ineinandergreifen. Während dieses Vorganges wird das Faservlies 6 auf das untere Transportband 17 gelegt, ohne dass die Fasern des Kopfendes 8 des aufgetrennten Faservlieses 6 umgelegt werden.
Nun kann das untere Transportband 17 und die Trommel 4 bewegt werden um das aufgetrennt Faservlies 6 von der Trommel 4 abzuziehen. Dabei wird auch das mittlere Transportband 24 des Einlegeschlittens 15 angetrieben. Die Welle 16 drückt über den Steg 23 die Transportbandachse 21 gegen die Transportbandachse 22, wodurch eine Klemmstelle für das Faservlies 6 entsteht, welche den Abziehvorgang des Vlieses 6 von der Trommel 4 unterstützt.

Für den Fall von wiederholten Bearbeitungsvorgängen (z.B. Parallelisierung und Verstreckung von Fasern) kann die erfindungsgemässe Auftrennvorrichtung das bearbeitete Faservlies 6 wiederholt von der gleichen Bearbeitungstrommel 4 abtrennen. Es ist auch möglich mit einer einzigen erfindungsgemässen Auftrennvorrichtung eine Mehrzahl von Bearbeitungstrommeln 4 zu bedienen, was die Konstruktion solcher Vorrichtungen vereinfacht und verbilligt.

Die erfindungsgemässen Auftrennvorrichtung kann vorteilhafterweise in einer Vorrichtung zur Herstellung von Faserbändern mit parallelisierter Faserlage aus Wirrfaserkollektiven verwendet werden, welche in der unter der Nummer EP-A-0 393 360 veröffentlichten europäischen Patentanmeldung beschrieben ist.

## Patentansprüche

1. Auftrennvorrichtung für auf Bearbeitungstrommeln aufgewickelte Faservliese (6) mit parallelisierter Faserlage mit einer Einlegevorrichtung (15-24), gekennzeichnet durch einen von und zur Bearbeitungstrommel (4) hin oszillierbar angeordneten Arm (1) mit einem Trennkamm (13), der längs einer Trennkurve (3) beweglich angeordnet ist, welche die Auftrennung des auf der Bearbeitungstrommel (4) aufgewickelten Faservlieses (6) an einer definierten Stelle und die Abwicklung des aufgetrennten Faservlieses von der Bearbeitungstrommel (4) mit dem einen freien Ende des Faservlieses (6) längs einer Mantelerzeugenden der Bearbeitungstrommel (4) bewirkt.

2. Auftrennvorrichtung nach Anspruch 1, dadurch gekennzeichnet, dass der Arm (1) aus einem Hebelwerk besteht mit mehreren, vorzugsweise drei gelenkig miteinander verbundenen Balken, wobei der Trennkamm (13) mit einem der Balken verbunden ist.

3. Auftrennvorrichtung nach Anspruch 1 oder 2, dadurch gekennzeichnet, dass der Arm (1) derart konstruiert ist, dass sich die Winkelstellung des Trennkammes (13) bezüglich der Bearbeitungstrommel (4) während der Bewegung des Armes (1) längs der Trennkurve (3) stetig ändert und den Mantel der Bearbeitungstrommel (4) tangential streift.

4. Auftrennvorrichtung nach einem der Ansprüche 1 bis 3, dadurch gekennzeichnet, dass die Winkelstellung des Arms (1) längs der Trennkurve (3) motorisch steuerbar ist.

5. Auftrennvorrichtung nach einem der Ansprüche 1 bis 4, dadurch gekennzeichnet, dass zusätzlich ein Niederhalter (5) vorgesehen ist um das Schleppende des Faservlieses (6) während des Trennvorganges an die Bearbeitungstrommel (4) pressen zu können.

6. Auftrennvorrichtung nach einem der Ansprüche 1 bis 5, dadurch gekennzeichnet, dass die Einlegevorrichtung (15-24) derart ausgebildet ist, dass das aufgetrennte Faservlies (6) mit den Fasern des Kopfendes (8), welche sich am Ende des Auftrennvorgangs in den Nadeln (2) befinden, einlegbar und von der Trommel (4) abziehbar ist.

7. Auftrennvorrichtung nach Anspruch 6, dadurch gekennzeichnet, dass die Einlegevorrichtung (15-24) einen Einlegeschlitten (15) umfasst, der mittels eines oberen Transportbandes (20) verfahrbar ist.

8. Auftrennvorrichtung nach Anspruch 7, dadurch gekennzeichnet, dass der Einlegeschlitten (15) ein mittleres Transportband (24) umfasst, welches über ein Zahnrad (18) antreibbar ist.

9. Auftrennvorrichtung nach Anspruch 8, dadurch gekennzeichnet, dass der Einlegeschlitten (15) zwischen einem rechten Anschlag des oberen Transportbandes (20), wo das aufgetrennte Faservlies (6) eingelegt wird, zu einem linken Anschlag des oberen Transportbandes (20) verfahrbar ist, wo das aufgetrennte Faservlies (6) zwischen zwei Transportbänder (17) eingelegt wird.

## Claims

1. A separation apparatus for separating fiber webs (6) wound on processing drums and with parallelized fiber positions, having an insertion device (15-24), characterized in that an arm (1) with a separation comb (13) is arranged in such a way that it can oscillate from and to the processing drum (4) and that the arm (1) is movably arranged along a separation curve (3) causing the separation of the fiber web (6) wound on the processing drum (4) at a specific site thereon and the unwinding of the separated fiber web from the processing drum (4) with one of the free ends of the fiber web (6) along a generatrix of the surface of the processing drum (4).

2. A separation apparatus according to claim 1, characterized in that the arm (1) comprises a lever apparatus having a plurality of, preferably three, mutually articulated links, the separation comb (13) being attached to one of said links.

3. A separation apparatus according to claim 1 or 2, characterized in that the arm (1) is designed in such a way that the angular position of the separation comb (13) with respect to the processing drum (4) continuously changes with motion of the arm (1) along the separation curve (3) and tangentially sweeps the surface of the processing drum (4).

4. A separation apparatus according to one of the claims 1 to 3, characterized in that the angular position of the arm (1) along the separation curve (3) is motor-controllable.

5. A separation apparatus according to one of the claims 1 to 4, characterized in that it further comprises retention means (5) to force the drag-end of the fiber web (6) against the processing drum (4) during the separation procedure.

6. A separation apparatus according to one of the claims 1 to 5, characterized in that the insertion device (15-24) is designed in such a way that the separated fiber web (6) can be inserted and drawn off from said processing drum (4) with its fibers of tip (8), which are located in the needles (2) after completion of the separation procedure.

7. A separation apparatus according to claim 6, characterized in that the insertion device (15-24) comprises an insertion sled (15) movable by an upper belt conveyor (20).

8. A separation apparatus according to claim 7, characterized in that the insertion sled (15) comprises a center belt conveyor (24) drivable by a toothed wheel (18).

9. A separation apparatus according to claim 8, characterized in that the insertion sled (15) is movable between a right end stop of the upper belt conveyor (20) at which said separated fiber web (6) is inserted and a left end stop of the upper belt conveyor (20) at which the separated fiber web (6) is inserted between two belt conveyors (17).

## Revendications

1. Dispositif de séparation pour feutres de fibres (6) à couches de fibres mises en parallèle, enroulés sur des tambours de traitement, avec un dispositif de pose (15-24), caractérisé en ce qu'un bras (1) avec un peigne de séparation (13) est disposé de manière à pouvoir osciller en va-et-vient par rapport au tambour de traitement (4), le bras (1) étant disposé de manière à se déplacer le long d'une courbe de séparation (3), qui réalise la séparation en un emplacement défini du feutre de fibre (6) enroulé sur le tambour de traitement (4), et qui effectue le déroulement hors du tambour de traitement (4) du feutre de fibres séparé, par une des extrémités libres du feutre de fibres (6), le long d'une génératrice de l'enveloppe du tambour de traitement (4).

2. Dispositif de séparation selon la revendication 1, caractérisé en ce que le bras (1) est constitué d'un mécanisme à levier avec plusieurs, de préférence trois, barreaux articulés l'un avec l'autre, le peigne de séparation (13) étant relié à un des barreaux.

3. Dispositif de séparation selon la revendication 1 ou 2, caractérisé en ce que le bras (1) est construit de telle sorte que la position angulaire du peigne de séparation (13) par rapport au tambour de traitement (4) se modifie en permanence pendant le déplacement du bras (1) le long de la courbe de séparation (3), et qu'il suit tangentiellement l'enveloppe du tambour de traitement (4).

4. Dispositif de séparation selon l'une des revendications 1 à 3, caractérisé en ce que la position angulaire du bras (1) le long de la courbe de séparation (3) peut être commandée à l'aide d'un moteur.

5. Dispositif de séparation selon l'une des revendications 1 à 4, caractérisé en ce qu'en plus un support inférieur (5) est prévu pour pouvoir repousser pendant l'opération de séparation l'extrémité traînante du feutre de fibres (6) contre le tambour de traitement (4).

6. Dispositif de séparation selon l'une des revendications 1 à 5, caractérisé en ce que le dispositif de pose (15-24) est configuré de telle sorte que le feutre de fibres (6) séparé puisse être posé et extrait du tambour (4) par les fibres de l'extrémité de tête (8) qui se trouvent dans les aiguilles (2) à la fin de l'opération de séparation.

7. Dispositif de séparation selon la revendication 6, caractérisé en ce que le dispositif de pose (15-24) comprend un traîneau d'insertion (15) pouvant être déplacé à l'aide d'une bande supérieure de transport (20).

8. Dispositif de séparation selon la revendication 7, caractérisé en ce que le traîneau d'insertion (15) comprend une bande médiane de transport (24) qui peut être entraînée par l'intermédiaire d'une roue dentée.

9. Dispositif de séparation selon la revendication 8, caractérisé en ce que le traîneau d'insertion (15) peut être déplacé entre une butée droite de la bande supérieure de transport (20), là où le feutre de fibres (6) séparé est inséré, et une butée de gauche de la bande supérieure de transport (20), là où le feutre de fibres (6) séparé est inséré entre deux bandes de transport (17).
